# EUROPEAN PATENT SPECIFICATION

(11) **EP 1 921 080 B1**
(45) Date of publication and mention of the grant of the patent: **05.08.2009**
(21) Application number: 06291725.7
(22) Date of filing: 07.11.2006
(51) Int. Cl.: C07D 487/04, A61K 31/519, A61P 25/28, A61P 35/00

(54) **Subsitituted 8-piperidinyl-2-pyridinyl-pyrimido(1,2-a)pyrimidin-6-one and 8-piperidinyl-2-pyrimidinyl-pyrimido(1,2-a)pyrimidin-6-one derivatives**
Subsitituierte Derivate des 8-Piperidinyl-2-pyridinylpyrimido(1,2-a)pyrimidin-6-ons und des 8-Piperidinyl-2-pyrimidinylpyrimido(1,2-a)pyrimidin-6-ons
Derivés substitués de 8-piperidinyl-2-pyridinyl-pyrimido(1,2-a)pyrimidin-6-one et de 8-piperidinyl-2-pyrimidinyl-pyrimido(1,2-a)pyrimidin-6-one

(43) Date of publication of application: 14.05.2008
(73) Proprietor: Sanofi-Aventis, 75013 Paris (FR); Mitsubishi Tanabe Pharma Corporation, Chuo-ku Osaka-shi (JP)
(72) Inventor: Lochead, Alistair W., Sanofi-Aventis, 92160 Antony (FR); Saady, Mourad., Sanofi-Aventis, 92160 Antony (FR); Slowinski, Frank, Sanofi-Aventis, 92160 Antony (FR); Yaïche, Philippe, Sanofi-Aventis, 92160 Antony (FR)
(74) Representative: Morel-Pécheux, Muriel

(56) References cited:
- EP-A- 1 557 417

## Description

### Technical Field

The present invention relates to compounds that are useful as an active ingredient of a medicament for preventive and/or therapeutic treatment of neurodegenerative diseases caused by abnormal activity of GSK3β.

### Background Art

GSK3β (glycogen synthase kinase 3β) is a proline directed serine, threonine kinase that plays an important role in the control of metabolism, differentiation and survival. It was initially identified as an enzyme able to phosphorylate and hence inhibit glycogen synthase. It was later recognized that GSK3β was identical to tau protein kinase 1 (TPK1), an enzyme that phosphorylates tau protein in epitopes that are also found to be hyperphosphorylated in Alzheimer's disease and in several taupathies. Interestingly, protein kinase B (AKT) phosphorylation of GSK3β results in a loss of its kinase activity, and it has been hypothesized that this inhibition may mediate some of the effects of neurotrophic factors. Moreover, phosphorylation by GSK3β of β-catenin, a protein involved in cell survival, results in its degradation by an ubiquitinilation dependent proteasome pathway.
Thus, it appears that inhibition of GSK3β activity may result in neurotrophic activity. Indeed there is evidence that lithium, an uncompetitive inhibitor of GSK3β, enhances neuritogenesis in some models and also increases neuronal survival, through the induction of survival factors such as Bcl-2 and the inhibition of the expression of proapoptotic factors such as P53 and Bax.
Recent studies have demonstrated that β-amyloid increases the GSK3β activity and tau protein phosphorylation. Moreover, this hyperphosphorylation as well as the neurotoxic effects of β-amyloid are blocked by lithium chloride and by a GSK3β antisense mRNA. These observations strongly suggest that GSK3β may be the link between the two major pathological processes in Alzheimer's disease: abnormal APP (Amyloid Precursor Protein) processing and tau protein hyperphosphorylation.

Although tau hyperphosphorylation results in a destabilization of the neuronal cytoskeleton, the pathological consequences of abnormal GSK3β activity are, most likely, not only due to a pathological phosphorylation of tau protein because, as mentioned above, an excessive activity of this kinase may affect survival through the modulation of the expression of apoptotic and antiapoptotic factors. Moreover, it has been shown that β-amyloid-induced increase in GSK3β activity results in the phosphorylation and, hence the inhibition of pyruvate dehydrogenase, a pivotal enzyme in energy production and acetylcholine synthesis.

Altogether these experimental observations indicate that GSK3β may find application in the treatment of the neuropathological consequences and the cognitive and attention deficits associated with Alzheimer's disease, as well as other acute and chronic neurodegenerative diseases. These include, in a nonlimiting manner, Parkinson's disease, tauopathies (e.g. frontotemporoparietal dementia, corticobasal degeneration, Pick's disease, progressive supranuclear palsy) and other dementia including vascular dementia; acute stroke and others traumatic injuries; cerebrovascular accidents (e.g. age related macular degeneration); brain and spinal cord trauma; peripheral neuropathies; retinopathies and glaucoma.

In addition GSK3β may find application in the treatment of other diseases such as: Non-insulin dependent diabetes (such as diabetes type II) and obesity; manic depressive illness; schizophrenia; alopecia; cancers such as breast cancer, non-small cell lung carcinoma, thyroid cancer, T or B-cell leukemia and several virus-induced tumors.

### Disclosure of the Invention

An object of the present invention is to provide compounds useful as an active ingredient of a medicament for preventive and/or therapeutic treatment of a disease caused by abnormal GSK3β activity, more particularly of neurodegenerative diseases. More specifically, the object is to provide novel compounds useful as an active ingredient of a medicament that enables prevention and/or treatment of neurodegenerative diseases such as Alzheimer's disease.

Thus, the inventors of the present invention have identified compounds possessing inhibitory activity against GSK3β. As a result, they found that compounds represented by the following formula (I) had the desired activity and were useful as an active ingredient of a medicament for preventive and/or therapeutic treatment of the aforementioned diseases.

The present invention thus provides as an object of the invention the pyrimidone derivatives represented by formula (I) or salts thereof, solvates thereof or hydrates thereof: 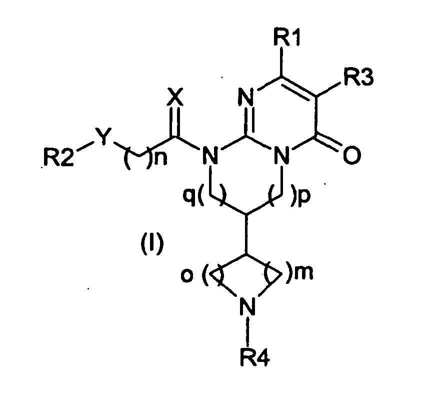 wherein:
X represents two hydrogen atoms, a sulfur atom, an oxygen atom or a C₁₋₂ alkyl group and a hydrogen atom;
Y represents a bond, a carbonyl group or a methylene group optionally substituted by one or two groups chosen from a C₁₋₆ alkyl group, a hydroxyl group, a C₁₋₆ alkoxy group, a C₁₋₂ perhalogenated alkyl group or an amino group;
R1 represents a 2, 3 or 4-pyridine ring or a 2, 4 or 5-pyrimidine ring, the ring being optionally substituted by a C₁₋₆ alkyl group, a C₁₋₆ alkoxy group or a halogen atom;
R2 represents a benzene ring or a naphthalene ring; the rings being optionally substituted by 1 to 4 substituents selected from a C₁₋₆alkyl group, a methylendioxy group, a halogen atom, a C₁₋₂ perhalogenated alkyl group, a C₁₋₃ halogenated alkyl group, a hydroxyl group, a C₁₋₆ alkoxy group, a nitro, a cyano, an amino, a C₁₋₆ monoalkylamino group or a C₂₋₁₂ dialkylamino group;
R3 represents a hydrogen atom, a C₁₋₆alkyl group or a halogen atom;
R4 represents a hydrogen atom, a C₁₋₆ alkoxy carbonyl group or a C₁₋₆ alkyl group optionally substituted by 1 to 4 substituents selected from a halogen atom, a phenyl group, a hydroxyl group or a C₁₋₆ alkoxy group;
n represents 0 to 3; p represents 2; q represents 0; o represents 0, 1 or 2 and m represents respectively 4, 3 or 2, o+m being equal to 4.

According to another aspect of the present invention, there is provided a medicament comprising as an active ingredient a substance selected from the group consisting of the pyrimidone derivatives represented by formula (I) and the physiologically acceptable salts thereof, and the solvates thereof and the hydrates thereof. As preferred embodiments of the medicament, there are provided the aforementioned medicament which is used for preventive and/or therapeutic treatment of diseases caused by abnormal GSK3β activity, and the aforementioned medicament which is used for preventive and/or therapeutic treatment of neurodegenerative diseases and in addition other diseases such as: Non-insulin dependent diabetes (such as diabetes type II) and obesity; manic depressive illness; schizophrenia; alopecia; cancers such as breast cancer, non-small cell lung carcinoma, thyroid cancer, T or B-cell leukemia and several virus-induced tumors.

As further embodiments of the present invention, there are provided the aforementioned medicament wherein the diseases are neurodegenerative diseases and are selected from the group consisting of Alzheimer's disease, Parkinson's disease, tauopathies (e.g. frontotemporoparietal dementia, corticobasal degeneration, Pick's disease, progressive supranuclear palsy) and other dementia including vascular dementia; acute stroke and others traumatic injuries; cerebrovascular accidents (e.g. age related macular degeneration); brain and spinal cord trauma; peripheral neuropathies; retinopathies and glaucoma, and the aforementioned medicament in the form of pharmaceutical composition containing the above substance as an active ingredient together with one or more pharmaceutical additives.

The present invention further provides an inhibitor of GSK3β activity comprising as an active ingredient a substance selected from the group consisting of the pyrimidone derivatives of formula (I) and the salts thereof, and the solvates thereof and the hydrates thereof.

According to further aspects of the present invention, there is provided a method for preventive and/or therapeutic treatment of neurodegenerative diseases caused by abnormal GSK3β activity, which comprises the step of administering to a patient a preventively and/or therapeutically effective amount of a substance selected from the group consisting of pyrimidone derivatives of formula (I) and the physiologically acceptable salts thereof, and the solvates thereof and the hydrates thereof; and a use of a substance selected from the group consisting of the pyrimidone derivatives of formula (I) and the physiologically acceptable salts thereof, and the solvates thereof and the hydrates thereof for the manufacture of the aforementioned medicament.
As used herein, the C₁₋₆ alkyl group represents a straight or branched alkyl group having 1 to 6 carbon atoms, for example, methyl group, ethyl group, n-propyl group, isopropyl group, n-butyl group, isobutyl group, sec-butyl group, tert-butyl group, n-pentyl group, isopentyl group, neopentyl group, 1,1-dimethylpropyl group, n-hexyl group, isohexyl group, and the like;
The C₁₋₆ alkoxy group represents an alkyloxy group having 1 to 4 carbon atoms for example, methoxy group, ethoxy group, propoxy group, isopropoxy group, butoxy group, isobutoxy group, sec-butoxy group, tert-butoxy group, and the like;
The halogen atom represents a fluorine, chlorine, bromine or iodine atom;
The C₁₋₂ perhalogenated alkyl group represents an alkyl group wherein all the hydrogen have been subsituted by a halogeno, for example a CF₃ or C₂F_{5;}
The C₁₋₃ halogenated alkyl group represents an alkyl group wherein at least one hydrogen has not been subsituted by an halogen atom;
The C₁₋₆ monoalkylamino group represents an amino group substituted by one C₁₋₆ alkyl group, for example, methylamino group, ethylamino group, propylamino group, isopropylamino group, butylamino group, isobutylamino group, tert-butylamino group, pentylamino group, isopentylamino group and the like;
The C₂₋₁₂ dialkylamino group represents an amino group substituted by two C₁₋₆ alkyl groups, for example, dimethylamino group, ethylmethylamino group, diethylamino group, methylpropylamino group and diisopropylamino group and the like;
A leaving group L represents a group which could be easily cleaved and substituted, such a group may be for example a tosyl, a mesyl, a bromide and the like.

The compounds represented by the aforementioned formula (I) may form a salt. Examples of the salt include, when an acidic group exists, salts of alkali metals and alkaline earth metals such as lithium, sodium, potassium, magnesium, and calcium; salts of ammonia and amines such as methylamine, dimethylamine, trimethylamine, dicyclohexylamine, tris(hydroxymethyl)aminomethane, *N,N-*bis(hydroxyethyl)piperazine, 2-amino-2-methyl-1-propanol, ethanolamine, *N-*methylglucamine, and L-glucamine; or salts with basic amino acids such as lysine, δ-hydroxylysine, and arginine. The base-addition salts of acidic compounds are prepared by standard procedures well known in the art.

When a basic group exists, examples include salts with mineral acids selected from hydrochloric acid, hydrobromic acid, sulfuric acid, nitric acid, phosphoric acid; salts with organic acids selected from methanesulfonic acid, benzenesulfonic acid, p-toluenesulfonic acid, acetic acid, propionic acid, tartaric acid, fumaric acid, maleic acid, malic acid, oxalic acid, succinic acid, citric acid, benzoic acid, mandelic acid, cinnamic acid, lactic acid, glycolic acid, glucuronic acid, ascorbic acid, nicotinic acid, and salicylic acid; or salts with acidic amino acids selected from aspartic acid, and glutamic acid.

The acid-addition salts of the basic compounds are prepared by standard procedures well know in the art which include, but are not limited thereto, dissolving the free base in an aqueous alcohol solution containing the appropriate acid and isolating the salt by evaporating the solution, or by reacting the free base and an acid in an organic solvent, in which case the salt separates directly, or is precipitated with a second organic solvent, or can be obtained by concentration of the solution. The acids which can be used to prepare the acid-addition salts are those which produce, when combined with the free base, pharmaceutically-acceptable salts, that is, salts whose anions are relatively innocuous to the animal organism in pharmaceutical doses of the salts, so that the beneficial properties inherent in the free base are not compromised by side effects ascribable to the anions.

In addition to the pyrimidone derivatives represented by the aforementioned formula (I) and salts thereof, their solvates and hydrates also fall within the scope of the present invention.

The pyrimidone derivatives represented by the aforementioned formula (I) may have one or more asymmetric carbon atoms. As for the stereochemistry of such asymmetric carbon atoms, they may independently be in either (R) and (S) configuration, and the derivative may exist as stereoisomers such as optical isomers, or diastereoisomers. Any stereoisomers in pure form, any mixtures of stereoisomers, racemates and the like fall within the scope of the present invention.

Examples of compounds of the present invention are shown in table 1 hereinafter. However, the scope of the present invention is not limited by these compounds.

An object of the present invention includes also compounds represented by formula (I) wherein X, m, n, o, p and q are as defined above and:
(1) R1 represents a 3- or 4-pyridine ring alternatively a 4- or 5-pyrimidine ring; the ring being optionally substituted by a C₁₋₂ alkyl group, a C₁₋₂ alkoxy group or a halogen atom; and/or
(2) R2 represents a benzene ring or a naphthalene ring; the ring being optionally substituted 1 to 4 substituents selected from a C₁₋₃ alkyl group, a halogen atom, a hydroxyl group or a C₁₋₂ alkoxy group; and/or
(3) R3 represents a hydrogen atom, a C₁₋₃ alkyl group or a halogen atom; and/or
(4) R4 represents a hydrogen atom, a C₁₋₄ alkoxy carbonyl group or a C₁₋₃ alkyl group optionally substituted by 1 to 4 substituents selected from a halogen atom, a hydroxyl group or a C₁₋₂ alkoxy group; and/or
(5) Y represents a bond, a carbonyl group or a methylene group optionally substituted by one or two groups chosen from a C₁₋₃ alkyl group, a hydroxyl group or a C₁₋₂ alkoxy group; and more particularly wherein R1, R2, R3, R4 and Y are as defined here-above.

Another object of the present invention includes compounds represented by formula (I) wherein n, p and q are as defined above and:
(1) R1 represents an unsubstituted 4-pyridine ring or 4-pyrimidine ring; more particularly an unsubstituted 4-pyridine ring; and/or
(2) R2 represents a benzene ring; the ring being optionally substituted 1 to 4 substituents selected from a C₁₋₃ alkyl group, a halogen atom, a hydroxyl group or a C₁₋₄ alkoxy group; and/or
(3) R3 represents a hydrogen atom; and/or
(4) R4 represents a hydrogen atom, a C₁₋₄ alkoxy carbonyl group or a C₁₋₃ alkyl group; and/or
(5) X represents two hydrogen atoms; and/or
(6) Y represents a carbonyl group or a methylene group optionally substituted by a hydroxyl group; and/or
(7) m represents 2 and o represents 2; and more particularly compounds wherein R1, R2, R3, R4, X, Y, p, m, o and q are as defined here-above.

A further object of the present invention includes the group of compound of formula as defined hereunder:
1. (+/-)1,1-Dimethylethyl4-[6-oxo-1-(2-oxo-2-phenylethyl)-8-(4-pyridinyl) -1,3,4,6-tetrahydro-2*H*-pyrimido[1,2-*a*]pyrimidin-2-yl]-1-piperidinecarboxylate
2. (+/-)Ethyl 4-[6-oxo-1-(2-oxo-2-phenylethyl)-8-(4-pyridinyl)-1 ,3,4,6-tetrahydro-2*H*-pyrimido[1,2-*a*]pyrimidin-2-yl]-1-piperidinecarboxylate
3. (+/-)9-(2-Oxo-2-phenylethyl)-8-(4-piperidinyl)-2-(4-pyridinyl)-6,7,8,9-tetrahydro-4H-pyrimido[1,2-*a*]pyrimidin-4-one
4. (+/-)8-(1-Methyl-4-piperidinyl)-9-(2-oxo-2-phenylethyl)-2-(4-pyridinyl)-6,7,8,9-tetrahydro-4*H*-pyrimido[1,2-*a*]pyrimidin-4-one
5. (+)9-(2-Oxo-2-phenylethyl)-8-(4-piperidinyl)-2-(4-pyridinyl)-6,7,8,9-tetrahydro-4*H*-pyrimido[1,2-*a*]pyrimidin-4-one
6. (-)9-(2-Oxo-2-phenylethyl)-8-(4-piperidinyl)-2-(4-pyridinyl)-6,7,8,9-tetrahydro-4*H*-pyrimido[1,2-*a*]pyrimidin-4-one
7. (+/-)9-[2-(4-fluoro-2-methoxy-phenyl)ethyl]-8-(4-piperidinyl)-2-(4-pyridinyl)-6,7,8,9-tetrahydro-4*H*-pyrimido[1,2-a]pyrimidin-4-one
8. (-)9-[2-Oxo-2-(3-bromo-phenyl)ethyl]-8-(4-piperidinyl)-2-(4-pyridinyl)-6,7,8,9-tetrahydro-4*H*-pyrimido[1,2-a]pyrimidin-4-one.

As a further object, the present invention concerns also methods for preparing the pyrimidone compounds represented by the aforementioned formula (I).

These compounds can be prepared, for example, according to methods explained below.

### Preparation method

Pyrimidone compounds represented by the aforementioned formula (I), may be prepared according to the method described in the scheme 1. 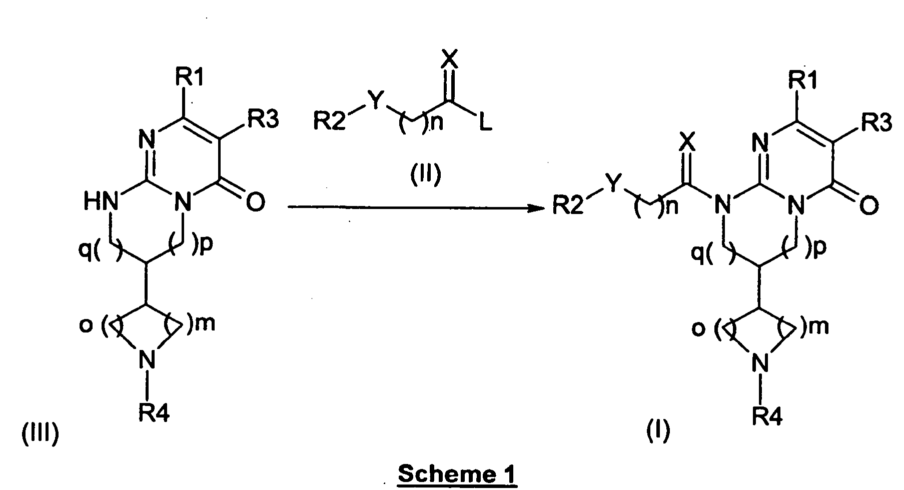 (In the above scheme the definition of R1, R2, R3, R4, X, Y, m, n, o, p and q are the same as those already described for compound of formula (I)).

Following this method, the pyrimidone derivative represented by the above formula (III), wherein R1, R3, R4, m, o, p and q are as defined for compound of formula (I), is allowed to react with a base such as sodium hydride, sodium carbonate or potassium carbonate in a solvent such as *N,N-*dimethylformamide, *N*-methylpyrrolidone, *N,N*-dimethylacetamide or chloroform at a suitable temperature ranging from 0 to 130°C under ordinary air, then with a compound of formula (II), wherein R2, X, Y and n are as defined for compound of formula (I) and L represents a leaving group preferably bromide or mesyl group, to obtain the compound of the aforementioned formula (I).

Alternatively compounds of formula (I) wherein Y represents a carbonyl group may be prepared by oxydation of a compound of formula (I) wherein Y represents a methylene group substituted by a hydroxyl group according to well known methods to one skilled in the art.

Compound of formula (II) is commercially available or may be synthesized according to well-known methods to one skilled in the art.

Compound of formula (III) may be prepared according to the method defined in scheme 2.

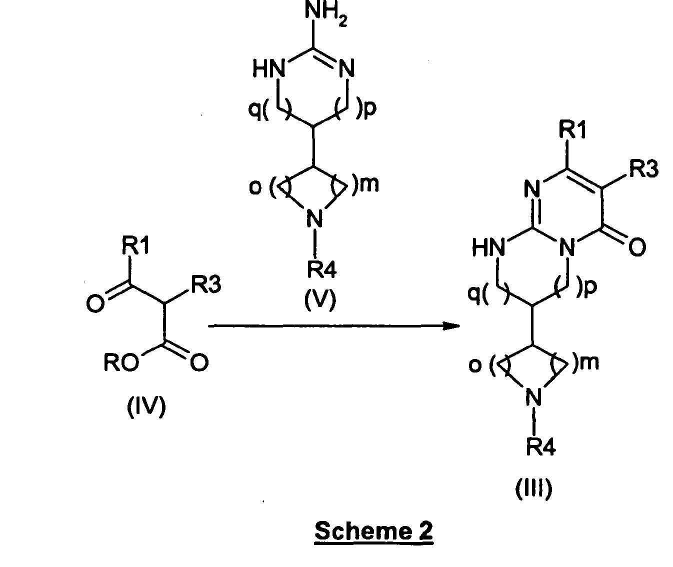

### (In the above scheme the definition of R1, R3, R4, m, o, p and q are the same as already described.)

According to this method, the 3-ketoester of formula (IV), wherein R1 and R3 are as defined for compound of formula (I) and R is an alkyl group such as for example methyl or ethyl, is allowed to react with a compound of formula (V) wherein R4, q and p are as defined for compound of formula (I). The reaction may be carried out in the presence of a base such as potassium carbonate, in an alcoholic solvent such as methanol, ethanol and the like or without, at a suitable temperature ranging from 25° to 140°C under ordinary air.

Alternatively, compound of formula (III), wherein R4 represents a hydrogen atom is allowed to react with a base such, sodium carbonate or triethylamine in a mixture of solvent such as tetrahydrofuran and water, then with a compound R4L of formula (VI),wherein R4 is as defined for compound of formula (I), beside the hydrogen atom, and L represents a leaving group preferably chloride, mesyl group or bromide, to give another compound of formula (III), wherein R4 is not a hydrogen atom.

Additionally compound of formula (III) wherein R3 represents a hydrogen atom may be halogenated in order to give compounds of formula (III) wherein R3 is a halogen atom such as a bromine atom or a chlorine atom. The reaction may be carried out in an acidic medium such as acetic acid or propionic acid, in presence of bromosuccinimide or chlorosuccimide, or bromine.

In addition, compounds of formula (III) wherein R3 represents a fluorine atom may be obtained by analogy to the method described in Tetrahedron Letters, Vol.30,No.45,pp 6113-6116, 1989.

As a further object, the present invention concerns also the compounds of formula (III) as intermediates of compounds of formula (I).

Compound of formula (IV) is commercially available or may be synthesized according to well-known methods to one skilled in the art.

For example compounds of formula (IV), wherein R1 represent a pyridine ring or a pyrimidine ring, optionally substituted by a C₁₋₆ alkyl group, C₁₋₆ alkoxy group or a halogen atom, can be prepared by reacting respectively an isonicotinic acid or a pyrimidine-carboxylic acid, optionally substituted by a C₁₋₆ alkyl group, C₁₋₆ alkoxy group or a halogen, with the corresponding malonic acid monoester. The reaction can be carried out using methods well known to one skilled in the art, such as for example in presence of a coupling agent such as 1,1'-carbonylbis-1*H-*imidazole in a solvent such as tetrahydrofuran at a temperature ranging from 20 to 70°C.

Compound of formula (V) may be synthesized according to well-known methods of one skilled in the art.

For example compound of formula (V), where p, q, m, o and R4 are as defined for compound of formula (I), may be prepared according to the method defined in scheme 3, starting from compound of formula (VIII). The conditions which may be used are given in the chemical examples.

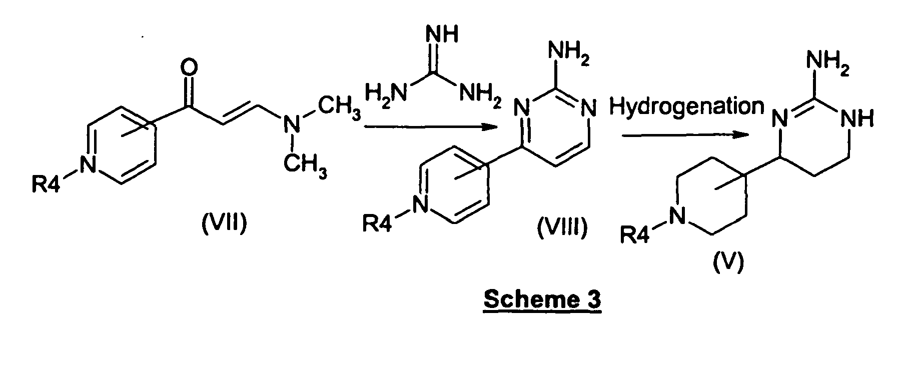

Compound of formula (VII) may be synthesized by analogy to the method described in J.Org.Chem. 1977, 42, 221-225.

Compound of formula (VIII) may be synthesized according to the method described in J. Med. Chem. 1978, 21, 623-628.

In the above reactions protection or deprotection of a functional group may sometimes be necessary. A suitable protecting group Pg can be chosen depending on the type of the functional group, and a method described in the literature may be applied. Examples of protecting groups, of protection and deprotection methods are given for example in Protective groups in Organic Synthesis Greene et al., 2nd Ed. (John Wiley & Sons, Inc., New York) 1985.

The compounds of the present invention have inhibitory activity against GSK3β. Accordingly, the compounds of the present invention are useful as an active ingredient for the preparation of a medicament, which enables preventive and/or therapeutic treatment of a disease caused by abnormal GSK3β activity and more particularly of neurodegenerative diseases such as Alzheimer's disease. In addition, the compounds of the present invention are also useful as an active ingredient for the preparation of a medicament for preventive and/or therapeutic treatment of neurodegenerative diseases such as Parkinson's disease, tauopathies (e.g. frontotemporoparietal dementia, corticobasal degeneration, Pick's disease, progressive supranuclear palsy) and other dementia including vascular dementia; acute stroke and others traumatic injuries; cerebrovascular accidents (e.g. age related macular degeneration); brain and spinal cord trauma; peripheral neuropathies; retinopathies and glaucoma; and other diseases such as non-insulin dependent diabetes (such as diabetes type II) and obesity; manic depressive illness; schizophrenia; alopecia; cancers such as breast cancer, non-small cell lung carcinoma, thyroid cancer, T or B-cell leukemia and several virus-induced tumors.

As the active ingredient of the medicament of the present invention, a substance may be used which is selected from the group consisting of the compound represented by the aforementioned formula (I) and pharmacologically acceptable salts thereof, and solvates thereof and hydrates thereof. The substance, per se, may be administered as the medicament of the present invention, however, it is desirable to administer the medicament in a form of a pharmaceutical composition which comprises the aforementioned substance as an active ingredient and one or more pharmaceutical additives. As the active ingredient of the medicament of the present invention, two or more of the aforementioned substances may be used in combination. The above pharmaceutical composition may be supplemented with an active ingredient of another medicament for the treatment of the above mentioned diseases. The type of pharmaceutical composition is not particularly limited, and the composition may be provided as any formulation for oral or parenteral administration. For example, the pharmaceutical composition may be formulated, for example, in the form of pharmaceutical compositions for oral administration such as granules, fine granules, powders, hard capsules, soft capsules, syrups, emulsions, suspensions, solutions and the like, or in the form of pharmaceutical compositions for parenteral administrations such as injections for intravenous, intramuscular, or subcutaneous administration, drip infusions, transdermal preparations, transmucosal preparations, nasal drops, inhalants, suppositories and the like. Injections or drip infusions may be prepared as powdery preparations such as in the form of lyophilized preparations, and may be used by dissolving just before use in an appropriate aqueous medium such as physiological saline. Sustained-release preparations such as those coated with a polymer may be directly administered intracerebrally.

Types of pharmaceutical additives used for the manufacture of the pharmaceutical composition, content ratios of the pharmaceutical additives relative to the active ingredient, and methods for preparing the pharmaceutical composition may be appropriately chosen by those skilled in the art. Inorganic or organic substances, or solid or liquid substances may be used as pharmaceutical additives. Generally, the pharmaceutical additives may be incorporated in a ratio ranging from 1 % by weight to 90% by weight based on the weight of an active ingredient.

Examples of excipients used for the preparation of solid pharmaceutical compositions include, for example, lactose, sucrose, starch, talc, cellulose, dextrin, kaolin, calcium carbonate and the like. For the preparation of liquid compositions for oral administration, a conventional inert diluent such as water or a vegetable oil may be used. The liquid composition may contain, in addition to the inert diluent, auxiliaries such as moistening agents, suspension aids, sweeteners, aromatics, colorants, and preservatives. The liquid composition may be filled in capsules made of an absorbable material such as gelatin. Examples of solvents or suspension mediums used for the preparation of compositions for parenteral administration, e.g. injections, suppositories, include water, propylene glycol, polyethylene glycol, benzyl alcohol, ethyl oleate, lecithin and the like. Examples of base materials used for suppositories include, for example, cacao butter, emulsified cacao butter, lauric lipid, witepsol.

The dose and frequency of administration of the medicament of the present invention are not particularly limited, and they may be appropriately chosen depending on conditions such as a purpose of preventive and/or therapeutic treatment, a type of a disease, the body weight or age of a patient, severity of a disease and the like. Generally, a daily dose for oral administration to an adult may be 0.01 to 1,000 mg (the weight of an active ingredient), and the dose may be administered once a day or several times a day as divided portions, or once in several days. When the medicament is used as an injection, administrations may preferably be performed continuously or intermittently in a daily dose of 0.001 to 100 mg (the weight of an active ingredient) to an adult.

### Chemical Examples

### Example 1 (Compound No. 1 of table 1)

### (+/-) 1,1-Dimethylethyl 4-[6-oxo-1-(2-oxo-2-phenylethyl)-8-(4-pyridinyl)-1,3,4,6-tetrahydro-2H-pyrimido[1,2-a]pyrimidin-2-yl]-1-piperidinecarboxylate

### 1.1 (+/-) 6-Piperidin-4-yl-1,4,5,6-tetrahydro-pyrimidin-2-ylamine (3:1) hydrochloride

To a solution of 5g (29.04 mmol) of 2-amino-4-(4-pyrimidyl)-pyrimidine (Journal of Medicinal Chemistry (1978), 21 (7), 623-8) in 30ml of a 6N solution of hydrochloric acid in isopropanol was added 5ml of water and 0.2g of palladium on carbon catalyst (10% wt/wt).
The suspension was hydrogenated under 40psi pressure at 50°C temperature during 8h.
The catalyst was removed by filtration and the solvent evaporated under reduced pressure. Isopropanol was added and the resulting solution was refiltered and the solvent removed by evaporation under reduced pressure to give 4.0g (55%) of compound as a white powder which was used as such.

### 1.2 (+/-)8-(4-Piperidinyl)-2-(4-pyridinyl)-6,7,8,9-tetrahydro-4H-pyrimido[1,2-a]pyrimidin-4-one

A mixture of 2.8g (14.50 mmol) of ethyl 3-(pyridin-4-yl)-3-oxopropionate, 3.7g (14.50 mmol) of (+/-)6-piperidin-4-yl-1,4,5,6-tetrahydro-pyrimidin-2-ylamine (3:1) hydrochloride and 6.01 g (43.50 mmol) of potassium carbonate in 50 ml of ethanol was heated at reflux temperature during 12 h.
The cooled solution was evaporated to remove solvent. The mixture was dissolved in dichloromethane, dried over sodium sulfate, evaporated, and the residue was chromatographed on silica gel eluting with a mixture of dichloromethane/methanol/aqueous ammonia solution (29%) in the proportions 100/0/0 to 80/20/2 led to afford 1.69g (37%) of the product as a white solid. Mp : 245-247°C.

### 1.3 (+/-)1,1-Dimethylethyl 4-[6-oxo-8-(4-pyridinyl)-1,3,4,6-tetrahydro-2H-pyrimido[1,2-a]pyrimidin-2-yl]-1-piperidinecarboxylate

To a solution of 1.7g (5.46 mmol) of (+/-)8-(4-piperidinyl)-2-(4-pyridinyl)-6,7,8,9-tetrahydro-4*H*-pyrimido[1,2-*a*]pyrimidin-4-one in 20 ml of tetrahydrofuran was added 11 ml of water, 0.761 ml (5.46 mmol) of triethylamine and 1.19g (5.46 mmol) of di-*tert*-butyl dicarbonate in 15 ml of tetrahydrofuran. The resulting mixture was stirred at room temperature for 2h. The mixture was evaporated to remove the solvent. The residue was dissolved in dichloromethane and washed with a saturated aqueous solution of ammonium chloride, saturated aqueous sodium chloride, dried over sodium sulfate and the solvent was evaporated. The crude product was recristallized from ethyl acetate and filtered to afford 2.19g (97%) of pure product as a white solid. Mp: 148-150°C.

### 1.4 (+/-)1,1-dimethylethyl 4-[6-oxo-1-(2-oxo-2-phenylethyl)-8-(4-pyridinyl)-1,3,4,6-tetrahydro-2H-pyrimido[1,2-a]pyrimidin-2-yl]-1-piperidinecarboxylate

To a solution of 0.31g (0.76 mmol) of (+/-)1,1-dimethylethyl 4-[6-oxo-8-(4-pyridinyl)-1,3,4,6-tetrahydro-2H-pyrimido[1,2-a]pyrimidin-2-yl]-1-piperidinecarboxylate in 6ml of anhydrous dimethylformamide was added 0.039g (0.98 mmol) of sodium hydride (60% suspension in mineral oil). The mixture was allowed to stir at 50°C for 1 h and cooled at 0°C. Then 0.18g (0.91 mmol) of 2-bromoacetophenone was added, the mixture allowed to stir at 0°C and at room temperature for 12h.
Water was added and the mixture allowed to stir at 0°C for 1 h. The precipitate was filtered and chromatographed on silica gel eluting with a mixture of chloroform/methanol in the proportions 100/0 to 97/3 to afford 0.140g of pure product as a white solid.
Mp : 179-181 °C.

### Example 2 (Compound No. 3 of table 1)

### (+/-)9-(2-Oxo-2-phenylethyl)-8-(4-piperidinyl)-2-(4-pyridinyl)-6,7,8,9-tetrahydro-4H-pyrimido[1,2-a]pyrimidin-4-one.

To a solution of 0.0847g (0.16 mmol) of (+/-)4-[6-oxo-1-(2-oxo-2-phenylethyl)-8-(4-pyridinyl)-1,3,4,6-tetrahydro-2*H*-pyrimido[1,2-*a*]pyrimidin-2-yl]-1-piperidinecarboxylate in 2 ml of tetrahydrofuran was added 5 ml of an aqueous solution of hydrochloric acid (2 N) and the resulting solution was stirred at room temperature for 2h.
The resulting mixture was evaporated and the residue was purified by chromatography on silica gel eluting with a mixture of dichloromethane/methanol/aqueous ammonia solution (29%) in the proportions 80/20/2 to afford 0.030g of pure product as a white solid.
Mp : 227-229 °C.

### Example 3 (Compound No. 2 of table 1)

### (+/-)Ethyl 4-[6-oxo-1-(2-oxo-2-phenylethyl)-8-(4-pyridinyl)-1,3,4,6-tetrahydro-2H-pyrimido[1,2-a]pyrimidin-2-yl]-1-piperidinecarboxylate.

### 3.1 (+/-)Ethyl 4-[6-oxo-8-(4-pyridinyl)-1,3,4,6-tetrahydro-2H-pyrimido[1,2-a]pyrimidin-2-yl]-1-piperidinecarboxylate.

To a solution of 0.2g (0.64 mmol) of (+/-)8-(4-piperidinyl)-2-(4-pyridinyl)-6,7,8,9-tetrahydro-4*H*-pyrimido[1,2-*a*]pyrimidin-4-one in 10 ml of dimethylformamide, 98µl (0.71 mmol) of triethylamine and 106µl (1.03 mmol) of ethyl chloroformate are added. The resulting mixture was stirred at room temperature for 16h.
Then the cooled mixture was added to dichloromethane and washed with a saturated aqueous solution of ammonium chloride followed by a saturated aqueous solution of sodium chloride. The organic solution was dried over sodium sulfate and evaporated to afford 0.2g of pure product as a yellow solid.
Mp : 185-187°C.

### 3.2 (+/-)Ethyl 4-[6-oxo-1-(2-oxo-2-phenylethyl)-8-(4-pyridinyl)-1,3,4,6-tetrahydro-2H-pyrimido[1,2-a]pyrimidin-2-yl]-1-piperidinecarboxylate.

The product was obtained by analogy with the method described in example 1 (step 1.4) and using (+/-)ethyl 4-[6-oxo-8-(4-pyridinyl)-1,3,4,6-tetrahydro-2*H-*pyrimido[1,2-*a*]pyrimidin-2-yl]-1-piperidinecarboxylate.
Mp : 159-161°C.

### Example 4 (Compound No. 4 of table 1)

### (+/-)8-(1-Methyl-4-piperidinyl)-9-(2-oxo-2-phenylethyl)-2-(4-pyridinyl)-6,7,8,9-tetrahydro-4H-pyrimido[1,2-a]pyrimidin-4-one. Hydrochloride (2:1).

### 4.1 (+/-)8-(1-Methyl-4-piperidinyl)-2-(4-pyridinyl)-6,7,8,9-tetrahydro-4H-pyrimido[1,2-a]pyrimidin-4-one

To a solution of 0.45g (1.45 mmol) of (+/-)8-(4-piperidinyl)-2-(4-pyridinyl)-6,7,8,9-tetrahydro-4*H*-pyrimido[1,2-*a*]pyrimidin-4-one, 0.452g (14.45 mmol) of paraformaldehyde in 10 ml of acetic acid was added 0.364g (6.99 mmol) of sodium cyanoborohydride. The resulting mixture was stirred at room temperature for 12h, the mixture was cooled at 0°C, then carefully poured in 60ml of an aqueous solution of sodium hydroxide (30%), extracted with dichloromethane, dried over sodium sulfate and evaporated. The residue was purified by chromatography on silica gel eluting with a mixture of dichloromethane/methanol/aqueous ammonia solution (29%) in the proportions 98/2/0.2 to 95/5/05 to afford 0.316g of pure product as an oil.

### 4.2 (+/-)8-(1-Methyl-4-piperidinyl)-9-(2-oxo-2-phenylethyl)-2-(4-pyridinyl)-6,7,8,9-tetrahydro-4H-pyrimido[1,2-a]pyrimidin-4-one. Hydrochloride (2:1).

The product was obtained by analogy with the method described in example 1 (step 1.4) and using (+/-)8-(1-Methyl-4-piperidinyl)-2-(4-pyridinyl)-6,7,8,9-tetrahydro-4*H*-pyrimido[1,2-*a*]pyrimidin-4-one.
Mp : 272-274°C.

### Example 5 (compound No.5 of table 1)

### (+)9-(2-Oxo-2-phenylethyl)-8-(4-piperidinyl)-2-(4-pyridinyl)-6,7,8,9-tetrahydro-4H-pyrimido[1,2-a]pyrimidin-4-one. Hydrochloride (2 :1)

117 mg (0.27 mmol) of (+/-)9-(2-Oxo-2-phenylethyl)-8-(4-piperidinyl)-2-(4-pyridinyl)-6,7,8,9-tetrahydro-4H-pyrimido[1,2-a]pyrimidin-4-one (compound No.3) was separated by chiral preparative HPLC (CHIRALCEL OD 250x50) eluting with isopropanol to give 0.046g of pure product obtained in the form of free base which was transformed into the hydrochloride salt. *t*_{R} : 56 min.
Mp : 269°C. [α]_{D}²⁰=+10.3° (c=0.724, ethanol).

### Example 6 (compound No.6 of table 1)

### (-)9-(2-Oxo-2-phenylethyl)-8-(4-piperinidyl)-2-(4-pyridinyl)-6,7,8,9-tetrahydro-4H-pyrimido[1,2-a]pyrimidin-4-one. Hydrochloride (2 :1)

117 mg (0.27 mmol) of (+/-)9-(2-Oxo-2-phenylethyl)-8-(4-piperinidyl)-2-(4-pyridinyl)-2-(4-pyridinyl)-6,7,8,9-tetrahydro-4H-pyrimido[1,2-a]pyrimidin-4-one (compound No.3) was separated by chiral preparative HPLC (CHIRALCEL OD 250x50) eluting with isopropanol to give 0.055g of pure product obtained in the form of free base which was transformed into the hydrochloride salt. *t*_{R} : 19 min. Mp : 269°C. [α]_{D}²⁰ = -15.7° (c=0.848, ethanol).

A list of chemical structures and physical data for compounds of the aforementioned formula (I), illustrating the present invention, is given in table 1. The compounds have been prepared according to the methods of the examples.

In the table, p, o and m represent 2, q represents 0, Et represents an ethyl group, Bu represents a butyl group, Ph represents a phenyl group, (Rot.) indicates the levorotatory or dextrorotatory properties of the enantiomeric compound.

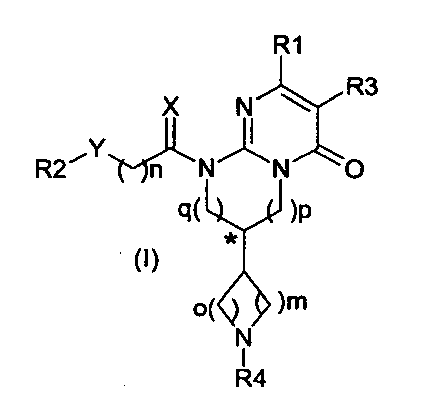

**Table 1**

| No. | Rot* | R2 | Y | X | R1 | R4 | R3 | n | Mp °C | salt |
|---|---|---|---|---|---|---|---|---|---|---|
| 1 | (+/-) | Ph | CO | H,H | 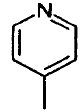 | CO₂tBu | H | 0 | 179-181 | Free base |
| 2 | (+/-) | Ph | CO | H,H | 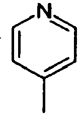 | CO₂Et | H | 0 | 159-161 | Free base |
| 3 | (+/-) | Ph | CO | H,H | 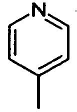 | H | H | 0 | 227-229 | Free base |
| 4 | (+/-) | Ph | CO | H,H | 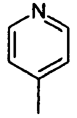 | CH₃ | H | 0 | 272-274 | (2:1) (hydrochloride) |
| 5 | (+) | Ph | CO | H,H | 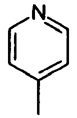 | H | H | 0 | 269 | (2:1) (hydrochloride) |
| 6 | (-) | Ph | CO | H,H | 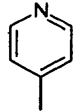 | H | H | 0 | 269 | (2:1) (hydrochloride) |
| 7 | (+/-) | 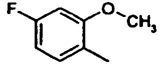 | bond | H,H | 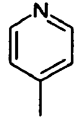 | H | H | 1 | 270-272 | Free base |
| 8 | (+/-) | 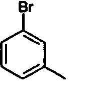 | CO | H,H | 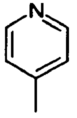 | H | H | 0 | 255-257 | (2:1) (hydrochloride) |

### Test Example: Inhibitory activity of the medicament of the present invention against GSK3β:

Two different protocols can be used.
In a first protocol : 7.5 µM of prephosphorylated GS1 peptide and 10 µM ATP (containing 300,000 cpm of 33P-ATP) were incubated in 25 mM Tris-HCl, pH 7.5, 0.6 mM DTT, 6 mM MgCl₂, 0.6 mM EGTA, 0.05 mg/ml BSA buffer for 1 hour at room temperature in the presence of GSK3beta (total reaction volume : 100 microliters).

In a second protocol : 4.1 µM of prephosphorylated GS1 peptide and 42 µM ATP (containing 260,000 cpm 33P-ATP) were incubated in 80 mM Mes-NaOH, pH 6.5, 1 mM Mg acetate, 0.5 mM EGTA, 5 mM 2-mercaptoethanol, 0.02% Tween 20, 10% glycerol buffer for 2 hours at room temperature in the presence of GSK3beta. Inhibitors were solubilised in DMSO (final solvent concentration in the reaction medium, 1 %).

The reaction was stopped with 100 microliters of a solution made of 25 g polyphosphoric acid (85% P₂O₅), 126 ml 85% H₃PO₄, H₂O to 500 ml and then diluted to 1:100 before use. An aliquot of the reaction mixture was then transferred to Whatman P81 cation exchange filters and rinsed with the solution described above. Incorporated 33P radioactivity was determined by liquid scintillation spectrometry.
The phosphorylated GS-1 peptide had the following sequence : NH2-YRRAAVPPSPSLSRHSSPHQS(P)EDEE-COOH.(Woodgett, J. R. (1989) Analytical Biochemistry 180, 237-241.

The GSK3β inhibitory activity of the compounds of the present invention are expressed in IC₅₀, and as an illustration the range of IC₅₀'s of the compounds in table 1 is between 10 nanomolar to 1 micromolar concentrations.
For example compound No. 3 of table 1 shows an IC₅₀ of 0.012µM.

### Formulation Example

### (1) Tablets

The ingredients below were mixed by an ordinary method and compressed by using a conventional apparatus.

| | |
|---|---|
| Compound of Example 1 | 30 mg |
| Crystalline cellulose | 60 mg |
| Corn starch | 100 mg |
| Lactose | 200 mg |
| Magnesium stearate | 4 mg |

### (2) Soft capsules

The ingredients below were mixed by an ordinary method and filled in soft capsules.

| | |
|---|---|
| Compound of Example 1 | 30 mg |
| Olive oil | 300 mg |
| Lecithin | 20 mg |

### (1) Parenteral preparations

The ingredients below were mixed by an ordinary method to prepare injections contained in a 1 ml ampoule.

| | |
|---|---|
| Compound of Example 1 | 3 mg |
| Sodium chloride | 4 mg |
| Distilled water for injection | 1 ml |

### Industrial Applicability

The compounds of the present invention have GSK3β inhibitory activity and are useful as an active ingredient of a medicament for preventive and/or therapeutic treatment of diseases caused by abnormal activity of GSK3β and more particularly of neurodegenerative diseases.

## Claims

1. A pyrimidone derivative represented by formula (I) or a salt thereof, or a solvate thereof or a hydrate thereof: 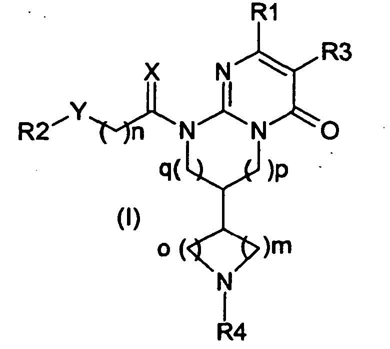 wherein:
X represents two hydrogen atoms, a sulfur atom, an oxygen atom or a C₁₋₂ alkyl group and a hydrogen atom;
Y represents a bond, a carbonyl group or a methylene group optionally substituted by one or two groups chosen from a C₁₋₆ alkyl group, a hydroxyl group, a C₁₋₆ alkoxy group, a C₁₋₂ perhalogenated alkyl group or an amino group;
R1 represents a 2, 3 or 4-pyridine ring or a 2, 4 or 5-pyrimidine ring, the ring being optionally substituted by a C₁₋₆ alkyl group, a C₁₋₆ alkoxy group, or a halogen atom; R2 represents a benzene ring or a naphthalene ring; the rings being optionally substituted by 1 to 4 substituents selected from a C₁₋₆ alkyl group, a methylendioxy group, a halogen atom, a C₁₋₂ perhalogenated alkyl group, a C₁₋₃ halogenated alkyl group, a hydroxyl group, a C₁₋₆ alkoxy group, a nitro, a cyano, an amino, a C₁₋₆ monoalkylamino group or a C₂₋₁₂ dialkylamino group;
R3 represents a hydrogen atom, a C₁₋₆ alkyl group or a halogen atom;
R4 represents a hydrogen atom, a C₁₋₆ alkoxy carbonyl group or a C₁₋₆ alkyl group optionally substituted by 1 to 4 substituents selected from a halogen atom, a phenyl group, a hydroxyl group or a C₁₋₆ alkoxy group;
n represents 0 to 3; p represents 2; q represents 0; o represents 0, 1 or 2 and m represents respectively 4, 3 or 2, o+m being equal to 4.

2. A pyrimidone derivative or a salt thereof, or a solvate thereof or a hydrate thereof according to claim 1, wherein R1 represents an unsubstituted 4-pyridine ring or unsubstituted 4-pyrimidine ring.

3. A pyrimidone derivative or a salt thereof, or a solvate thereof or a hydrate thereof according to claim 1 or 2, wherein
• R1 represents an unsubstituted 4-pyridine ring or 4-pyrimidine ring;
• R2 represents a benzene ring; the ring being optionally substituted 1 to 4 substituents selected from a C₁₋₃ alkyl group, a halogen atom, a hydroxyl group or a C₁₋₂ alkoxy group;
• R3 represents a hydrogen atom;
• R4 represents a hydrogen atom, a C₁₋₄ alkoxy carbonyl group or a C₁₋₃ alkyl group;
• X represents two hydrogen atoms;
• Y represents a carbonyl group or a methylene group optionally substituted by a hydroxyl group;
• p represents 2, m represents 2, o represents 2 and q represents 0.

4. A pyrimidone derivative which is selected from the group consisting of:
• (+/-)1,1-Dimethylethyl 4-[6-oxo-1-(2-oxo-2-phenylethyl)-8-(4-pyridinyl)-1,3,4,6-tetrahydro-2*H-*pyrimido[1,2*-a*]pyrimidin-2-yl]-1-piperidinecarboxylate
• (+/-)Ethyl 4-[6-oxo-1-(2-oxo-2-phenylethyl)-8-(4-pyridinylyl,3,4,6-tetrahydro-2*H-*pyrimido[1,2*-a*]pyrimidin-2-yl]-1-piperidinecarboxylate
• (+/-)9-(2-Oxo-2-phenylethyl)-8-(4-piperidinyl)-2-(4-pyridinyl)-6,7,8,9-tetrahydro-4*H-*pyrimido[1,2*-a*]pyrimidin-4-one
• (+/-)8-(1-Methyl-4-piperidinyl)-9-(2-oxo-2-phenylethyl)-2-(4-pyridinyl)-6,7,8,9-tetrahydro-4*H-*pyrimido[1,2-*a*]pyrimidin-4-one
• (+)9-(2-Oxo-2-phenylethyl)-8-(4-piperidinyl)-2-(4-pyridinyl)-6,7,8,9-tetrahydro-4*H-*pyrimido[1,2*-a*]pyrimidin-4-one
• (-)9-(2-Oxo-2-phenylethyl)-8-(4-piperidinyl)-2-(4-pyridinyl)-6,7,8,9-tetrahydro-4*H-*pyrimido[1,2*-a*]pyrimidin-4-one
• (+/-)9-[2-(4-fluoro-2-methoxy-phenyl)ethyl]-8-(4-piperidinyl)-2-(4-pyridinyl)-6,7,8,9-tetrahydro-4*H*-pyrimido[1,2*-a*]pyrimidin-4-one or
• (-)9-[2-Oxo-2-(3-bromo-phenyl)ethyl]-8-(4-piperidinyl)-2-(4-pyridinyl)-6,7,8,9-tetrahydro-4*H-*pyrimido[1,2-a]pyrimidin-4-one, or a salt thereof, or a solvate thereof or a hydrate thereof.

5. A compound of formula (III) 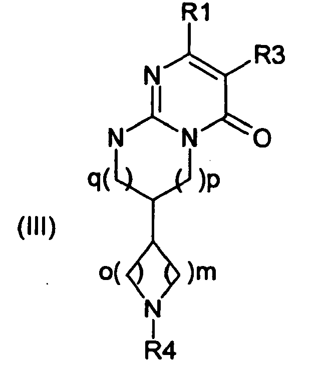 wherein R1, R3, R4, m, o, p and q are as defined for compound of formula (I) according to claim 1.

6. A medicament comprising as an active ingredient a substance selected from the group consisting of pyrimidone derivative represented by formula (I) or salts thereof, or a solvate thereof or a hydrate thereof according to claim 1.

7. A GSK3β inhibitor selected from the group of a pyrimidone derivative represented by formula (I) or salts thereof, or a solvate thereof or a hydrate thereof according to claim 1.

8. Use of a compound according to claims 1 to 4 for the preparation of a medicament for preventive and/or therapeutic treatment of a neurodegenerative disease.

9. Use of a compound according to claim 8, wherein the neurodegenerative disease is selected from the group consisting of Alzheimer's disease, Parkinson's disease, tauopathies, vascular dementia; acute stroke, traumatic injuries; cerebrovascular accidents, brain cord trauma, spinal cord trauma; peripheral neuropathies; retinopathies or glaucoma.

10. Use of a compound according to claims 1 to 4 for the preparation of a medicament for preventive and/or therapeutic treatment of non-insulin dependent diabetes; obesity; manic depressive illness; schizophrenia; alopecia; or cancers.

11. Use according to claim 10 wherein cancer is breast cancer, non-small cell lung carcinoma, thyroid cancer, T or B-cell leukemia or virus-induced tumors.

## Patentansprüche

1. Pyrimidonderivate der Formel (I) und deren Salze und deren Solvate und deren Hydrate: 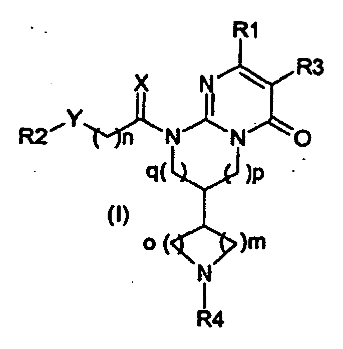 wobei:
X für zwei Wasserstoffatome, ein Schwefelatom, ein Sauerstoffatom oder eine C₁₋₂-Alkylgruppe und ein Wasserstoffatom steht;
Y für eine Bindung, eine Carbonylgruppe oder eine Methylengruppe, gegebenenfalls substituiert durch eine oder zwei Gruppen ausgewählt aus einer C₁₋₆-Alkylgruppe, einer Hydroxylgruppe, einer C₁₋₆-Alkoxygruppe, -einer perhalogenierten C₁₋₂-Alkylgruppe oder einer Aminogruppe, steht;
R1 für einen 2-, 3- oder 4-Pyridinring oder einen 2-, 4- oder 5-Pyrimidinring steht, wobei der Ring gegebenenfalls durch eine C₁₋₆-Alkylgruppe, eine C₁₋₆-Alkoxygruppe oder ein Halogenatom substiutiert ist;
R2 für einen Benzolring oder einen Naphthalinring steht, wobei die Ringe gegebenenfalls durch 1 bis 4 Substituenten ausgewählt aus einer C₁₋₆-Alkylgruppe, einer Methylendioxygruppe, einem Halogenatom, einer perhalogenierten C₁₋₂-Alkylgruppe, einer halogenierten C₁₋₃-Alkylgruppe, einer Hydroxylgruppe, einer C₁₋₆-Alkoxygruppe, einer Nitrogruppe, einer Cyanogruppe, einer Aminogruppe, einer C₁₋₆-Monoalkylaminogruppe oder einer C₂₋₁₂-Dialkylaminogruppe substituiert sind;
R3 für ein Wasserstoffatom, eine C₁₋₆-Alkylgruppe oder ein Halogenatom steht;
R4 für ein Wasserstoffatom, eine C₁₋₆-Alkoxycarbonylgruppe oder eine C₁₋₆-Alkylgruppe, gegebenenfalls substituiert durch 1 bis 4 Substituenten ausgewählt aus einem Halogenatom, einer Phenylgruppe, einer Hydroxylgruppe oder einer C₁₋₆-Alkoxygruppe, steht;
n für 0 bis 3 steht; p für 2 steht; q für 0 steht; o für 0, 1 oder 2 steht und m für 4, 3 bzw. 2 steht, wobei o+m gleich 4 ist.

2. Pyrimidonderivate und deren Salze und deren Solvate und deren Hydrate nach Anspruch 1, wobei R1 für einen unsubstituierten 4-Pyridinring oder einen unsubstituierten 4-Pyrimidinring steht.

3. Pyrimidonderivate und deren Salze und deren Solvate und deren Hydrate nach Anspruch 1 oder 2, wobei
• R1 für einen unsubstituierten 4-Pyridinring oder 4-Pyrimidinring steht;
• R2 für einen Benzolring steht, wobei der Ring gegebenenfalls durch 1 bis 4 Substituenten ausgewählt aus einer C₁₋₃-Alkylgruppe, einem Halogenatom, einer Hydroxylgruppe oder einer C₁₋₂-Alkoxygruppe substituiert ist;
• R3 für ein Wasserstoffatom steht;
• R4 für ein Wasserstoffatom, eine C₁₋₄-Alkoxycarbonylgruppe oder eine C₁₋₃-Alkylgruppe steht;
• X für zwei Wasserstoffatome steht;
• Y für eine Carbonylgruppe oder eine Methylengruppe, gegebenenfalls substituiert durch eine Hydroxylgruppe, steht;
• p für 2 steht, m für 2 steht, o für 2 steht und q für 0 steht.

4. Pyrimidonderivate, ausgewählt aus der Gruppe bestehend aus:
• (+/-)-4-[6-Oxo-1-(2-oxo-2-phenylethyl)-8-(4-pyridinyl)-1,3,4,6-tetrahydro-2*H*-pyrimido[1,2-a]pyrimidin-2-yl]-1-piperidincarbonsäure-1,1-dimethylethylester;
• (+/-)-4-[6-Oxo-1-(2-oxo-2-phenylethyl)-8-(4-pyridinyl)-1,3,4,6-tetrahydro-2*H*-pyrimido[1,2-a]pyrimidin-2-yl]-1-piperidincarbonsäure-ethylester;
• (+/-)-9-(2-Oxo-2-phenylethyl)-8-(4-piperidinyl)-2-(4-pyridinyl)-6,7,8,9-tetrahydro-4*H*-pyrimido[1,2-a]pyrimidin-4-on;
• (+/-)-8-(1-Methyl-4-piperidinyl)-9-(2-oxo-2-phenylethyl)-2-(4-pyridinyl)-6,7,8,9-tetrahydro-4*H-*pyrimido[1,2-*a*]pyrimidin-4-on;
• (+)-9-(2-Oxo-2-phenylethyl)-8-(4-piperidinyl)-2-(4-pyridinyl)-6,7,8,9-tetrahydro-4*H-*pyrimido[1,2-*a*]pyrimidin-4-on;
• (-)-9-(2-Oxo-2-phenylethyl)-8-(4-piperidinyl)-2-(4-pyridinyl)-6,7,8,9-tetrahydro-4*H-*pyrimido[1,2-*a*]pyrimidin-4-on;
• (+/-)-9-[2-(4-Fluor-2-methoxyphenyl)ethyl]-8-(4-piperidinyl)-2-(4-pyridinyl)-6,7,8,9-tetrahydro-4*H-*pyrimido[1,2*-a*]pyrimidin-4-on oder
• (-)-9-[2-Oxo-2-(3-bromphenyl)ethyl]-8-(4-piperidinyl)-2-(4-pyridinyl)-6,7,8,9-tetrahydro-4*H-*pyrimido[1,2*-a*]pyrimidin-4-on und deren Salzen und deren Solvaten und deren Hydraten.

5. Verbindungen der Formel (III) 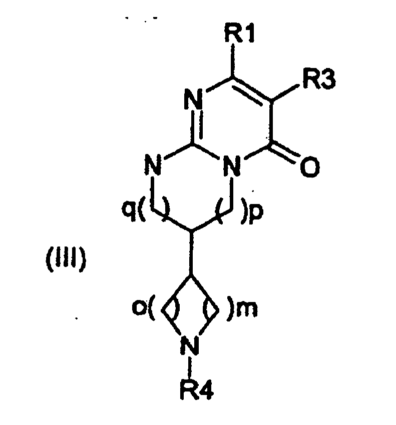 wobei R1, R3, R4, m, o, p und q wie für Verbindungen der Formel (I) nach Anspruch 1 definiert sind.

6. Medikament, welches als Wirkstoff eine Substanz ausgewählt aus der aus den durch die Formel (I) wiedergegebenen Pyrimidonderivaten und deren Salzen und deren Solvaten und deren Hydraten nach Anspruch 1 bestehenden Gruppe.

7. GSK3β-Inhibitor, ausgewählt aus der aus durch die Formel (I) wiedergegebenen Pyrimidonderivaten und deren Salzen und deren Solvaten und deren Hydraten nach Anspruch 1 bestehenden Gruppe.

8. Verwendung einer Verbindung nach einem Ansprüche 1 bis 4 zur Herstellung eines Medikaments zur präventiven und/oder therapeutischen Behandlung einer neurodegenerativen Krankheit.

9. Verwendung einer Verbindung nach Anspruch 8, wobei die neurodegenerative Krankheit ausgewählt ist aus der Gruppe bestehend aus Alzheimer-Krankheit, Parkinson-Krankheit, Tauopathien, vaskulärer Demenz, akutem Schlaganfall, traumatischen Verletzungen, Schlaganfällen, Hirn-Rückenmark-Trauma, Rückenmarktrauma, peripheren Neuropathien, Retinopathien und Glaukom.

10. Verwendung einer Verbindung nach einem der Ansprüche 1 bis 4 zur Herstellung eines Medikaments zur präventiven und/oder therapeutischen Behandlung von nichtinsulinabhängiger Diabetes, Obesitas, manischdepressiver Krankheit, Schizophrenie, Alopecie oder Krebs.

11. Verwendung nach Anspruch 10, wobei es sich bei dem Krebs um Brustkrebs, nichtkleinzelliges Lungenkarzinom, Schildrüsenkrebs, T- oder B-Zellen-Leukämie oder virusinduzierte Tumore handelt.

## Revendications

1. Dérivé de pyrimidone représenté par la formule (I) ou sel de celui-ci, ou solvate de celui-ci ou hydrate de celui-ci : 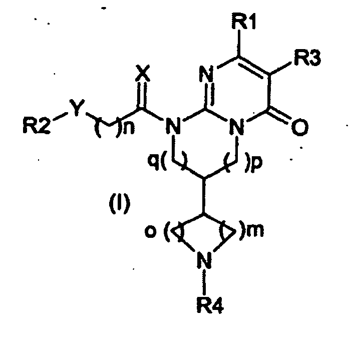 dans laquelle :
X représente deux atomes d'hydrogène, un atome de soufre, un atome d'oxygène ou un groupement alkyle en C₁₋₂ et un atome d'hydrogène ;
Y représente une liaison, un groupement carbonyle ou un groupement méthylène éventuellement substitué par un ou deux groupements choisis parmi un groupement alkyle en C₁₋₆, un groupement hydroxy, un groupement alcoxy en C₁₋₆, un groupement alkyle perhalogéné en C₁₋₂ ou un groupement amino ;
R1 représente un cycle 2, 3 ou 4-pyridine ou un cycle 2, 4 ou 5-pyrimidine, le cycle étant éventuellement substitué par un groupement alkyle en C₁₋₆, un groupement alcoxy en C₁₋₆ ou un atome d'halogène ;
R2 représente un cycle benzène ou un cycle napthalène ; les cycles étant éventuellement substitués par 1 à 4 substituants choisis parmi un groupement alkyle en C₁₋₆, un groupement méthylènedioxy, un atome d'halogène, un groupement alkyle perhalogéné en C₁₋₂ , un groupement alkyle halogéné en C₁₋₃, un groupement hydroxy, un groupement alcoxy en C₁₋₆, un nitro, un cyano, un amino, un groupement monoalkylamino en C₁₋₆ ou un groupement dialkylamino en C₂₋₁₂ ;
R3 représente un atome d'hydrogène, un groupement alkyle en C₁₋₆ ou un atome d'halogène ;
R4 représente un atome d'hydrogène, un groupement alcoxycarbonyle en C₁₋₆ ou un groupement alkyle en C₁₋₆ éventuellement substitué par 1 à 4 substituants choisis parmi un atome d'halogène, un groupement phényle, un groupement hydroxy ou un groupement alcoxy en C₁₋₆;
n représente 0 à 3 ; p représente 2 ; q représente 0 ; o représente 0, 1 ou 2 et m représente respectivement 4, 3 ou 2, o+m étant égal à 4.

2. Dérivé de pyrimidone ou sel de celui-ci, ou solvate de celui-ci ou hydrate de celui-ci selon la revendication 1, **caractérisé en ce que** R1 représente un cycle 4-pyridine non substitué ou un cycle 4-pyrimidine non substitué.

3. Dérivé de pyrimidone ou sel de celui-ci, ou solvate de celui-ci ou hydrate de celui-ci selon la revendication 1 ou 2, **caractérisé en ce que**
• R1 représente un cycle 4-pyridine ou 4-pyrimidine non substitué ;
• R2 représente un cycle benzène ; le cycle étant éventuellement substitué par 1 à 4 substituants choisis parmi un groupement alkyle en C₁₋₃, un atome d'halogène, un groupement hydroxy ou un groupement alcoxy en C₁₋₂ ;
• R3 représente un atome d'hydrogène ;
• R4 représente un atome d'hydrogène, un groupement alcoxycarbonyle en C₁₋₄ ou un groupement alkyle en C₁₋₃ ;
• X représente deux atomes d'hydrogène ;
• Y représente un groupement carbonyle ou un groupement méthylène éventuellement substitué par un groupement hydroxy ;
• p représente 2, m représente 2, o représente 2 et q représente 0.

4. Dérivé de pyrimidone, **caractérisé en ce qu'**il est choisi dans le groupe constitué de :
• 4-[6-oxo-1-(2-oxo-2-phényléthyl)-8-(4-pyridinyl)-1,3,4,6-tétrahydro-2*H-*pyrimido[1,2-a]pyrimidin-2-yl]-1-pipéridinecarboxylate de (+/-)1,1-diméthyléthyle
• 4-[6-oxo-1-(2-oxo-2-phényléthyl)-8-(4-pyridinyl)-1,3,4,6-tétrahydro-2*H-*pyrimido[1,2-a]pyrimidin-2-yl]-1-pipéridinecarboxylate de (+/-)éthyle
• (+/-)9-(2-oxo-2-phényléthyl)-8-(4-pipéridinyl)-2-(4-pyridinyl)-6,7,8,9-tétrahydro-4*H-*pyrimido[1,2*-a*]pyrimidin-4-one
• (+/-)8-(1-méthyl-4-pipéridinyl)-9-(2-oxo-2-phényléthyl)-2-(4-pyridinyl)-6,7,8,9-tétrahydro-4*H-*pyrimido[1,2*-a*]pyrimidi-n-4-one
• (+)9-(2-oxo-2-phényléthyl)-8-(4-pipéridinyl)-2-(4-pyridinyl)-6,7,8,9-tétrahydro-4*H-*pyrimido[1,2*-a*]pyrimidin-4-one
• (-)9-(2-oxo-2-phényléthyl)-8-(4-pipéridinyl)-2-(4-pyridinyl)-6,7,8,9-tétrahydro-4*H-*pyrimido[1,2*-a*]pyrimidin-4-one
• (+/-)9-[2-(4-fluoro-2-méthoxy-phényl)éthyl]-8-(4-pipéridinyl)-2-(4-pyridinyl)-6,7,8,9-tétrahydro-4*H-*pyrimido[1,2*-a*]pyrimidin-4-one ou
• (-)9-[2-oxo-2-(3-bromo-phényl)éthyl]-8-(4-pipéridinyl)-2-(4-pyridinyl)-6,7,8,9-tétrahydro-4*H-*pyrimido[1,2*-a*]pyrimidin-4-one, ou sel de ceux-ci, ou solvate de ceux-ci ou hydrate de ceux-ci.

5. Composé de formule (III) 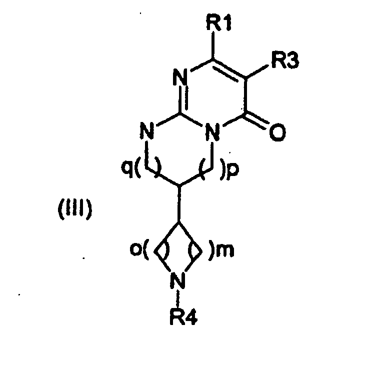 dans laquelle R1, R3, R4, m, o, p et q sont tels que définis pour le composé de formule (I) selon la revendication 1.

6. Médicament comprenant comme principe actif une substance choisie dans le groupe constitué d'un dérivé de pyrimidone représenté par la formule (I) ou des sels de celui-ci, ou d'un solvate de celui-ci ou d'un hydrate de celui-ci selon la revendication 1.

7. Inhibiteur de GSK3β choisi dans le groupe de dérivé de pyrimidone représenté par la formule (I) ou de sels de celui-ci, ou de solvate de celui-ci ou d'hydrate de celui-ci selon la revendication 1.

8. Utilisation d'un composé selon les revendications 1 à 4, pour la préparation d'un médicament destiné au traitement préventif et/ou thérapeutique d'une maladie neurodégénérative.

9. Utilisation d'un composé selon la revendication 8, **caractérisée en ce que** la maladie neurodégénérative est choisie dans le groupe constitué de la maladie d'Alzheimer, de la maladie de Parkinson, des tauopathies, de la démence vasculaire ; des attaques aiguës, des lésions traumatiques ; des accidents vasculaires cérébraux, des traumatismes cérébraux, des traumatismes médullaires ; des neuropathies périphériques ; des rétinopathies ou du glaucome.

10. Utilisation d'un composé selon les revendications 1 à 4, pour la préparation d'un médicament destiné au traitement préventif et/ou thérapeutique du diabète non insulino-dépendent ; de l'obésité ; de la psychose maniaco-dépressive ; de la schizophrénie ; de l'alopécie ; ou de cancers.

11. Utilisation selon la revendication 10, **caractérisée en ce que** le cancer est le cancer du sein, le carcinome du poumon non à petites cellules, le cancer de la thyroïde, la leucémie des lymphocytes T ou B ou les tumeurs induites par des virus.
